# EUROPEAN PATENT SPECIFICATION

(11) **EP 3 795 897 B1**
(45) Date of publication and mention of the grant of the patent: **18.12.2024**
(21) Application number: 18918884.0
(22) Date of filing: 15.05.2018
(51) Int. Cl.: H05B 47/10, F21V 21/40, F21V 23/04, F21W 131/205, F21V 14/00, A61B 90/30, G06F 3/01

(54) **CONTROL METHOD FOR SURGICAL LIGHT, AND SURGICAL LIGHT**
STEUERVERFAHREN FÜR OPERATIONSLEUCHTE UND OPERATIONSLEUCHTE
PROCÉDÉ DE COMMANDE DE LUMIÈRE CHIRURGICALE, ET LUMIÈRE CHIRURGICALE

(43) Date of publication of application: 24.03.2021
(73) Proprietor: Nanjing Mindray Bio-Medical Electronics Co., Ltd., Nanjing, Jiangsu 211111 (CN)
(72) Inventor: WANG, Lei, Nanjing, Jiangsu (CN)
(74) Representative: KIPA AB
(86) International application number: PCT/CN2018/086877
(87) International publication number: WO 2019/218153

(56) References cited:
- WO-A1-2013/178781
- WO-A1-2017/103794
- CN-A- 102 374 464
- CN-A- 106 813 170
- CN-A- 107 448 822
- CN-A- 107 846 763
- CN-A- 107 847 284
- CN-A- 107 850 290
- US-A1- 2010 053 982
- US-A1- 2012 043 915
- US-A1- 2012 043 915
- US-A1- 2017 296 291

## Description

### Technical Field

The present application relates to the field of medical instruments, and in particular to a method for controlling a surgical light, and a surgical light.

### Background Art

Surgical lights provide good illumination conditions for surgeons to perform an operation. For the observation effect during the operation, the surgeon will hope to manually adjust the position and illumination state of the surgical light by himself according to the actual needs. In addition, during the operation, both hands of the surgeon are in a sterile state and are strictly forbidden to touch non-sterile articles, so the light body of the surgical light is usually provided with an aseptic handle that has been sterilized. At present, only some of the functions of the surgical light can be adjusted by the aseptic handle, and the operation is cumbersome.

US 2012/0043915 A1 describes operating light has a housing, which has a light exit opening; a holding system, which is coupled with housing and is designed to movably hold the operating light. A grip assembly unit is provided approximately in the middle of light exit opening. The grip assembly unit has a handle to pivot the operating light into a desired position. At least one rotating ring is located between the handle and the housing and is rotatable in relation to the handle and is designed to set various functions of operating light. As an alternative or in addition, a touch sensitive sensor element, which is designed to set various functions of operating light, is located between the handle and the housing.

US 2017/0296291 A1 discloses a light head comprising a plurality of light sources for generating a light field on a site to be illuminated and a method for modifying features of said light field.

### Summary of the Invention

Embodiments of the present application provide a method for controlling a surgical light, and the surgical light, which can adjust an illumination parameter corresponding to a holding gesture when a user operates an aseptic handle.

In a first aspect, an embodiment of the present application provides a method for controlling a surgical light, the method comprising: obtaining holding information when a user holds an aseptic handle, the holding information including holding gestures and/or holding positions, and different holding gestures and/or different holding positions corresponding to different illumination parameters; determining an illumination parameter corresponding to the holding information according to the obtained holding information; obtaining an adjustment instruction triggered by the user; and adjusting the illumination parameter corresponding to the holding information according to the adjustment instruction.

In a second aspect, an embodiment of the present application provides a surgical light, comprising: a light body, an aseptic handle, a detection circuit, and a control circuit, wherein the light body comprises a shell and an illumination apparatus installed in the shell; the detection circuit is used to obtain holding information when a user holds the aseptic handle, the holding information including holding gestures and/or holding positions, and different holding gestures and/or different holding positions corresponding to different illumination parameters, and obtain adjustment instruction triggered by the user; and the control circuit is used to determine an illumination parameter corresponding to the holding information according to the obtained holding information, and adjust the illumination parameter according to the adjustment instruction obtained by the detection circuit.

According to the technical solution provided by the embodiments of the present application, the adjustment of the illumination parameter corresponding to the holding information is achieved by means of obtaining holding information when a user holds an aseptic handle, determining an illumination parameter corresponding to the holding information according to the obtained holding information, obtaining an adjustment instruction triggered by the user, and adjusting the illumination parameter corresponding to the holding information according to the adjustment instruction. The operation is convenient, and the user experience is improved.

### Brief Description of the Drawings

Fig. 1A is a schematic diagram of a holding gesture when a user holds an aseptic handle.
Fig. 1B is a schematic diagram of another holding gesture when the user holds the aseptic handle.
Fig. 2A is a schematic structural diagram of a surgical light according to an embodiment of the present application.
Fig. 2B is a schematic structural diagram of an aseptic handle according to an embodiment of the present application.
Fig. 3A is a schematic diagram showing the case when a user holds an aseptic handle according to an embodiment of the present application.
Fig. 3B is a distance characteristic graph corresponding to the holding gesture shown in Fig. 3A.
Fig. 3C is a schematic diagram showing the case when a user holds an aseptic handle according to another embodiment of the present application.
Fig. 3D is a distance characteristic graph corresponding to the holding gesture shown in Fig. 3C.
Fig. 4A is a schematic diagram showing the case when a user holds an aseptic handle according to an embodiment of the present application.
Fig. 4B is a schematic diagram of a planar contour corresponding to the holding gesture shown in Fig. 4A.
Fig. 4C is a schematic diagram of a linear contour corresponding to the holding gesture shown in Fig. 4A.
Fig. 4D is a schematic diagram showing the case when a user holds an aseptic handle according to an embodiment of the present application.
Fig. 4E is a schematic diagram of a planar contour corresponding to the holding gesture shown in Fig. 4D.
Fig. 4F is a schematic diagram of a linear contour corresponding to the holding gesture shown in Fig. 4D.
Fig. 5A is a schematic diagram showing a user holding, in a gesture, an aseptic handle having a region recognition mark.
Fig. 5B is a schematic diagram showing a user holding, in another gesture, an aseptic handle having a region recognition mark.
Fig. 6A is a schematic diagram showing a user holding, in a gesture, an aseptic handle having a location notch.
Fig. 6B is a schematic diagram showing a user holding, in another gesture, an aseptic handle having a location notch.
Fig. 7A is a schematic diagram showing a user holding an aseptic handle in a gesture.
Fig. 7B is a schematic diagram showing a user holding, in another gesture, an aseptic handle having a location notch.
Fig. 7C is a schematic diagram showing a user holding an aseptic handle at different positions in a gesture shown in Fig. 7B.
Fig. 8A is a schematic diagram showing a user holding an aseptic handle in a gesture.
Fig. 8B is a schematic diagram showing a user holding an aseptic handle at different positions in a gesture shown in Fig. 8A.
Fig. 8C is a schematic diagram showing a user holding an aseptic handle in another gesture.

### Detailed Description of Embodiments

The embodiments of the present application will be described below in conjunction with the drawings in the embodiments of the present application.

Embodiments of the present application provide a method for controlling a surgical light, and the surgical light, which can adjust an illumination parameter corresponding to a holding gesture when a user operates an aseptic handle, improving the user experience.

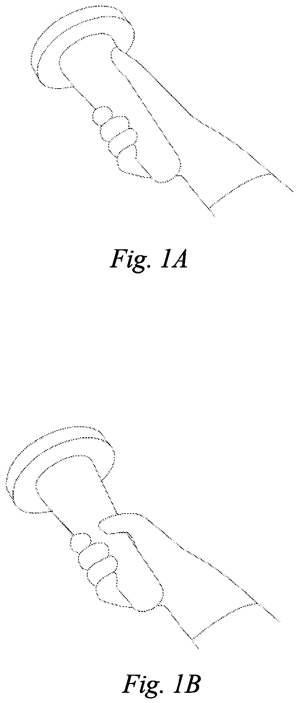

People usually take two gestures when holding an article, such as a rod or a handle. With holding an aseptic handle as an example, two gestures shown in Figs. 1A and 1B are included. The holding gesture in Fig. 1A is a gesture that the thumb axially extends along the aseptic handle when the user holds the aseptic handle. The holding gesture in Fig. 1B is a gesture that the thumb radially extends along the aseptic handle when the user holds the aseptic handle. The two gestures are often used in daily life. For example, in sports such as badminton, the gesture of holding a racket is usually as shown in Fig. 1B, which is more flexible when hitting the ball in the frontcourt and midcourt; and when hitting the ball in the backcourt, in order to increase the hitting force, the holding gesture as shown in Fig. 1A is usually used to hit the ball. People can consciously switch between the two holding gestures as needed at any time in sports. There are many similar scenes in daily life. Therefore, the two holding gestures are both commonly used and can be actively and clearly distinguished. It should be understood that a surgeon may also use the two holding gestures when holding an aseptic handle. Different illumination parameters are set to correspond to different holding gestures. During the operation, the controlled illumination parameters are switched by means of changing the holding gesture, and the illumination parameter corresponding to the holding gesture is adjusted by means of changing the direction of rotation of the aseptic handle.

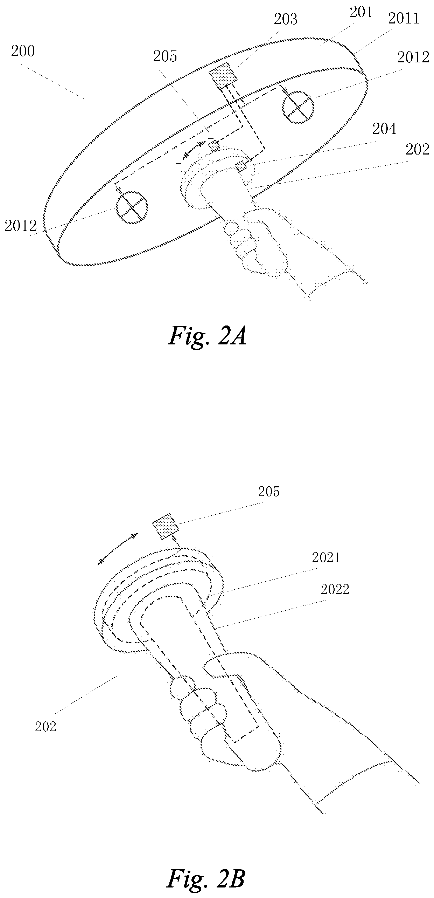

Referring to Fig. 2A, Fig. 2A is a schematic structural diagram of a surgical light 200. As shown in Fig. 2A, the surgical light 200 comprises: a light body 201, an aseptic handle 202, a control circuit 203, a first detection circuit 204, and a second detection circuit 205, wherein the light body 201 comprises a shell 2011, and an illumination apparatus 2012 installed in the shell 2011.

The aseptic handle 202 is connected to the light body 201 for holding by a user, and the aseptic handle 202 can rotate relative to the light body 201. The surgical light 200 further comprises a detection circuit that comprises a first detection circuit 204 and a second detection circuit 205.

The first detection circuit 204 is used to obtain the holding information that the user holds the aseptic handle 202, and the control circuit 203 can determine the illumination parameter corresponding to the current holding information according to the holding information. In some possible implementation manners of the present invention, the holding gestures corresponding to the holding information may include: a first preset holding gesture (the gesture that the thumb axially extends along the aseptic handle), and a second preset holding gesture (the gesture that the thumb radially extends along the aseptic handle).

In some possible implementation manners of the present application, the illumination parameter corresponding to the first preset holding information may be set to be a diameter of illumination spot of an illumination apparatus in the surgical light, and the illumination parameter corresponding to the second preset holding information may be set to be a illumination brightness of the illumination apparatus in the surgical light. It should be understood that the correspondence relationship between the preset holding information and the illumination parameter can be set and adjusted as needed. The illumination parameter controlled by the current operation is determined according to the current holding information. For example, if the current holding information is the first preset holding information, the illumination parameter controlled by the current operation is the diameter of illumination spot. If the current holding information is the second preset holding information, the illumination parameter controlled by the current operation is the illumination brightness.

The second detection circuit 205 is used to obtain an adjustment instruction triggered by the user. In some possible implementation manners of the present application, the second detection circuit 205 is used to obtain rotation information of the aseptic handle 202, with the rotation state indicated by the rotation information including clockwise rotation or counterclockwise rotation. The trigger instruction obtained by the second detection circuit 205 during the clockwise rotation may be set to be the adjustment instruction of increasing the illumination parameter, and the trigger instruction obtained by the second detection circuit 205 during the counterclockwise rotation is the adjustment instruction of decreasing the illumination parameter. Of course, in some embodiments, the trigger instruction obtained by the second detection circuit 205 during the clockwise rotation may also be set to be the adjustment instruction of decreasing the illumination parameter, and the trigger instruction obtained by the second detection circuit 205 during the counterclockwise rotation is an adjustment instruction of increasing the illumination parameter. It should be understood that, the time when the second detection circuit 205 obtains the adjustment instruction triggered by the user and the time when the control circuit 203 determines, according to the holding information, the illumination parameter corresponding to the current holding information are not in a particular order. They can be performed simultaneously, or may be performed in the order in which at first the second detection circuit 205 obtains the adjustment instruction triggered by the user, or may be performed in the order in which the control circuit 203 determines the illumination parameter corresponding to the current holding according to the holding information.

The control unit 203 is used to adjust the illumination parameter corresponding to the holding information obtained by the first detection circuit 204 according to the adjustment instruction obtained by the second detection circuit 205. The illumination parameter may include: a diameter of illumination spot, an illumination brightness, or a color temperature, etc. For example, taking the example in which the illumination parameter is increased during the clockwise rotation and the illumination parameter is decreased during the counterclockwise rotation, if the current holding information is the second preset holding information and the aseptic handle is operated to rotate clockwise, the control unit 203 controls, according to the current operation, the illumination apparatus to enhance the illumination brightness.

It should be noted that the specific structure of the aseptic handle 202 may be as shown in Fig. 2B, comprising an inner handle 2021 and an outer handle 2022 sheathed over the inner handle 2021. The outer handle 2022 may be disposable, or may be sterilized for reuse.

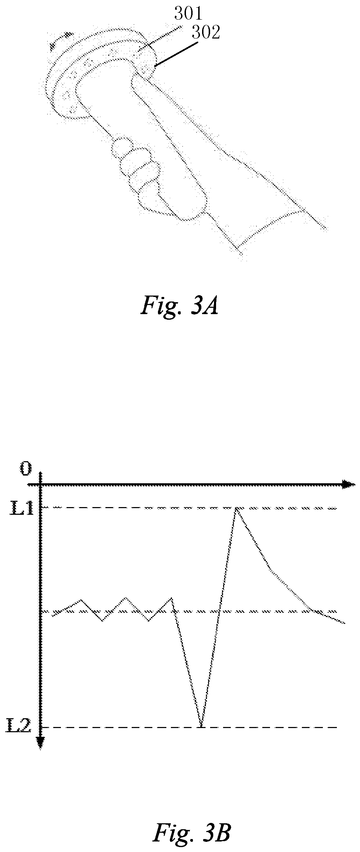

Referring to Fig. 3A, this is a schematic diagram showing the case when a user holds an aseptic handle. The first detection circuit comprises: first processor (not shown in Fig. 3A) and multiple distance sensors 301. The multiple distance sensors 301 may be evenly arranged around the top edge of the aseptic handle, and specifically, the distance sensors 301 may be arranged on the shell of the light holder and located at the edge of the top of the aseptic handle. It should be noted that, if the top of the aseptic handle comprises a protruding ring-shaped structure 302, the distance sensors 301 can be located either on the outer edge of the ring-shaped structure 302 on the shell or, as shown in Fig. 3A, on the inner side of the ring-shaped structure 302, and specifically, the distance sensors 301 may be located either on the shell or on the inner handle. When the distance sensors 301 are located on the shell, the aseptic handle may be provided with through holes respectively at positions corresponding to the distance sensors or is provided with a hollowed ring corresponding to all the distance sensors so as to facilitate signal transmission of the distance sensors. When the distance sensors 301 are located on the inner handle of the aseptic handle, the outer handle may be provided with through holes at positions corresponding to the distance sensors or is provided with a hollowed ring corresponding to all the distance sensors.

The distance sensor may be an optical distance sensor, an infrared distance sensor, or an ultrasonic distance sensor, etc., which is not limited here. Taking the optical distance sensor as an example, when the user holds the aseptic handle, the light emitted by the optical distance sensor reflects back from the hand, and the optical distance sensor determines the distance from the hand by means of measuring the difference in time between emission and reception. A distance characteristic graph connecting the distance values can be obtained according to a distance values from the hand detected by the distance sensors, and then the difference between the maximum value and the minimum value in the distance characteristic graph. It should be understood that, when a thumb is axially placed along the aseptic handle, the difference between the maximum value and the minimum value in the distance characteristic graph is larger. When a thumb is radially placed along the aseptic handle, the difference between the maximum value and the minimum value in the distance characteristic graph is smaller. When the current holding information is determined, if the difference is greater than L0, it is determined that the current holding information is the first preset holding information. If the difference is less than or equal to L0, it is determined that the current holding information is the second preset holding information.

For example, when the gesture that the user holds the aseptic handle is the gesture that, as shown in Fig. 3A, the thumb axially extends along the aseptic handle, the obtained holding information is the first preset holding information, and the distance characteristic graph obtained according to the distance sensors is as shown in Fig. 3B, with the turning point and starting point in Fig. 3B respectively corresponding to vertical distances from different distance sensors to the hand, in which the minimum value is L1, and the maximum value is L2. (L2 - L1) > L0, so it can be determined that the current holding information is the first preset holding information.

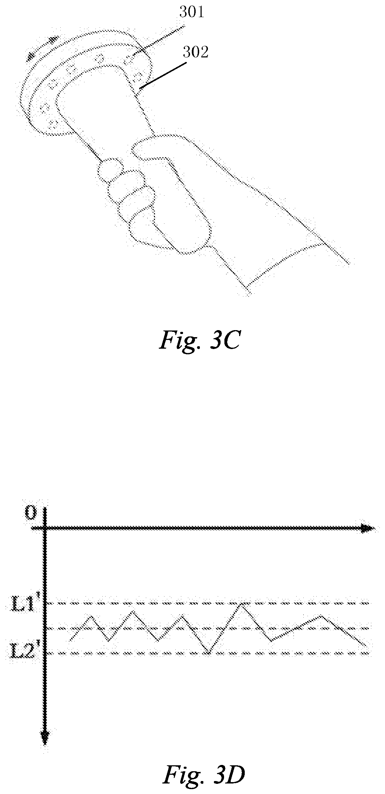

When the gesture that the user holds the aseptic handle is as shown in Fig. 3C, the distance characteristic graph obtained by the sensors is as shown in Fig. 3D, with the turning point and starting point in Fig. 3D respectively corresponding to the vertical distances from different distance sensors to the hand, in which the minimum value is L1', and the maximum value is L2'. (L2' - L1') < L0, so it can be determined that the current holding information is the second preset holding information.

The holding information may also be determined by the sensors provided on the surface of the aseptic handle. Specifically, the contour information of the hand when the user holds the aseptic handle can be obtained by the sensors, the current holding information of the user can be determined according to the contour information, and then it is determined which type of preset holding information that the holding information is according to the current holding information. It should be understood that, when the structure of the aseptic handle, as shown in Fig. 2B, comprises an inner handle and an outer handle sheathed over the inner handle, the sensors can be arranged at the outer surface of the inner handle. Specifically, the whole outer surface of the inner handle may be provided with sensors, or only part of the outer surface of the inner handle is provided with sensors, as long as recognizing different holding gestures.

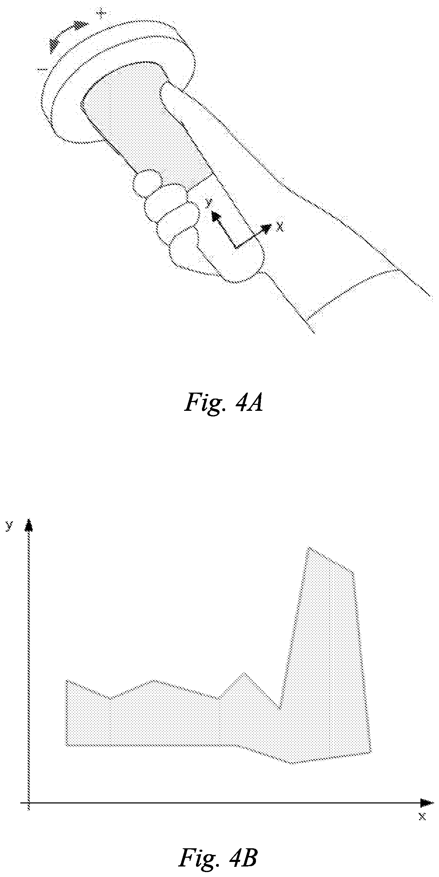

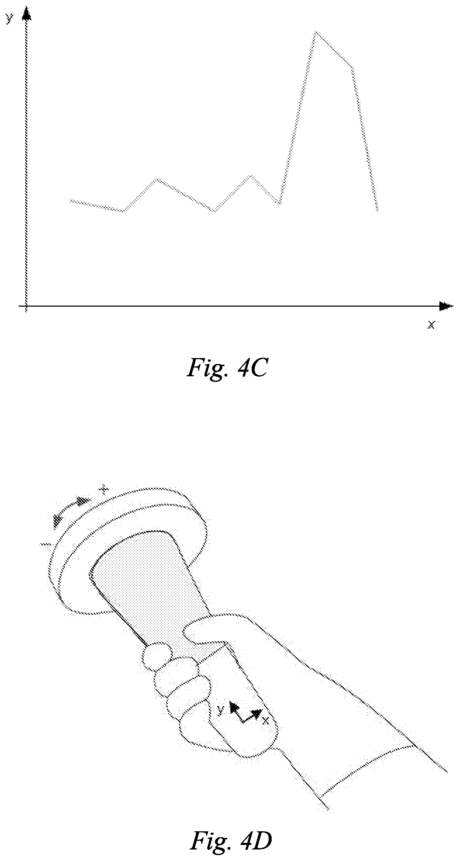

Taking the example in which the sensors are provided in the region of upper half portion of the outer surface of the inner handle, when the gesture that the user holds the aseptic handle is, as shown in Fig. 4A, the holding gesture corresponding to the first preset holding information, that is, the thumb being axially placed along the aseptic handle. In some possible implementation manners, the sensors (which may be pressure sensors) detect that the contour that the hand comes into contact with the sensors is a planar contour, as shown in Fig. 4B. In other possible implementation manners, the sensors (which may be pressure sensors) may detect that the contour that the hand comes into contact with the sensors is a linear contour, as shown in Fig. 4C. It is determined which type of preset holding information that the current holding information is according to the planar contour or the linear contour obtained by the sensors. For example, the holding gesture that the vertical distance between the lowest point and the highest point of the contour is greater than LO can be set to be the first preset holding information, and the holding information that the vertical distance between the lowest point and the highest point is less than or equal to LO can be set to be the second preset holding gesture. Corresponding to Figs. 4B and 4C, if the vertical distance between the lowest point and the highest point of the contour is greater than L0, it is determined that the current holding information is the first preset holding information.

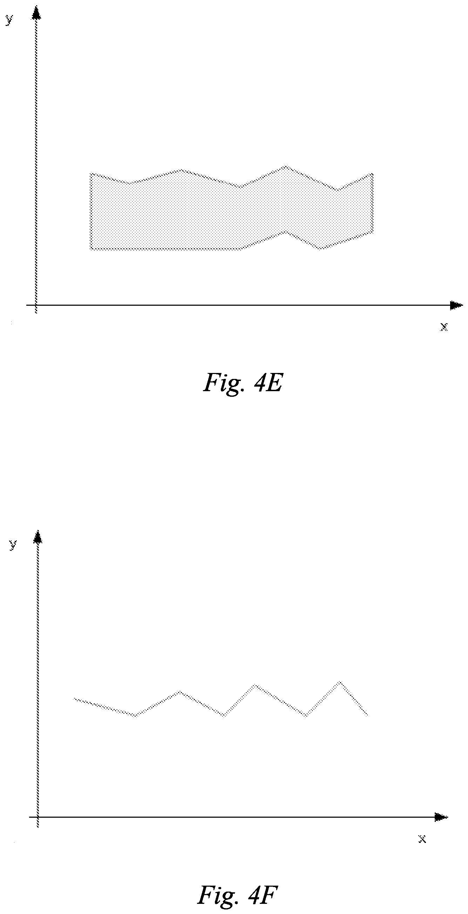

When the gesture that the user holds the aseptic handle, as shown in Fig. 4D, corresponds to the second preset holding information, the thumb radially extends along the aseptic handle, that is, the thumb and the index finger form a ring-shape. In some possible implementation manners, the sensors can detect that the contour that the hand comes into contact with the sensors is a planar contour, as shown in Fig. 4E. In other possible implementation manners, the sensors can detect that the contour that the hand comes into contact with the sensors is a linear contour, as shown in Fig. 4F. Corresponding to Figs. 4E and 4F, if the vertical distance between the lowest point and the highest point of the contour is less than L0, it is determined that the current holding information is the second preset holding information.

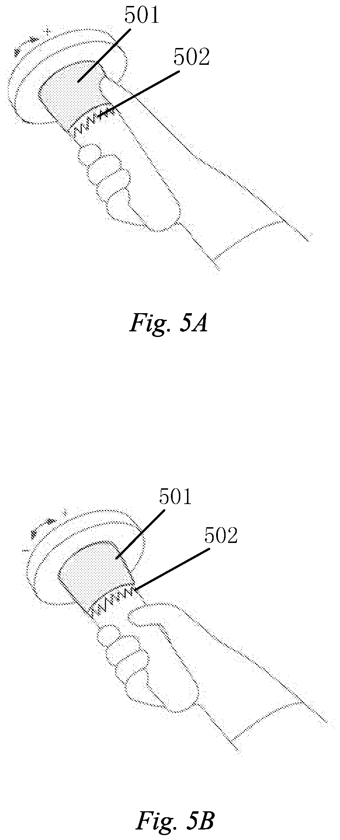

As an improvement, in some possible implementation manners of the present application, the aseptic handle may be provided with a recognition region to detect the user's touch signal on the recognition region and determine the holding information according to the touch signal. The outer surface of the aseptic handle may also be provided with a raised structure, or a groove structure, or a convex-concave alternating structure, by which the aseptic handle is divided into different regions, for example, the region above the structures is determined as the recognition region. As shown in Figs. 5A and 5B, in the figures, the outer surface of the aseptic handle is provided with a serrated sensible ring structure 502, which may be raised, or may be recessed, or may be a convex-concave alternating structure. When the specific structure of the aseptic handle is as shown in Fig. 2B, the sensible region recognition mark may be arranged at the outer surface of the outer handle. The first detection circuit 501 may be located above the sensible structure 502 and on the outer surface of the inner handle of the aseptic handle, as the region corresponding to the gray background in Fig. 5A.

According to the embodiment of the present application, by means of providing the recognition region, during operation, the holding information corresponding to the operation that a finger is located in the recognition region is determined as the first preset holding information, the holding information corresponding to the operation that no finger is located in the recognition region is determined as the second preset holding information, and providing the sensible region recognition mark is convenient for the user to easily recognize and change the holding information and thus changes the adjusted illumination parameter.

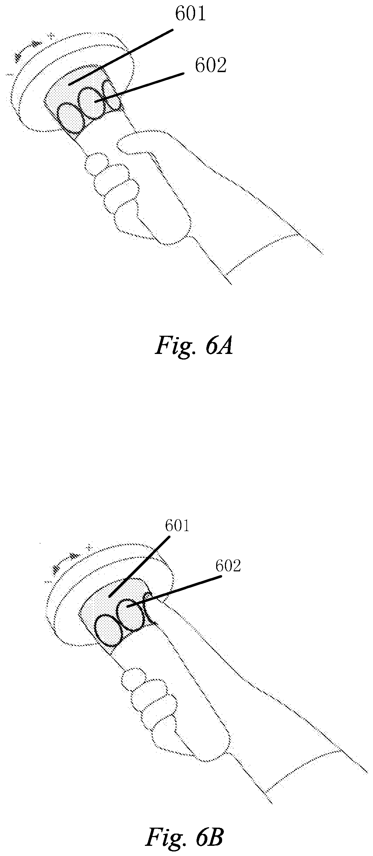

As an improvement, in some possible implementation manners of the present application, the aseptic handle may be provided with a recognition region and a location region, and the detection circuit obtains the user's touch signals in the recognition region and the location region and determines the holding information according to the touch signal. For example, at least one location notch may be circumferentially provided on the upper portion of the aseptic handle, as the structure indicated by 602 in Fig. 6A. When it is detected that the user's finger moves to any location notch, the first detection circuit determines that the current holding information is the first preset holding information. When the user's finger is not on any location notch, the first detection circuit determines that the current holding gesture is the second holding gesture.

When the specific structure of the aseptic handle is as shown in Fig. 2B, the location notch 602 may be arranged at the outer surface of the outer handle. The first detection circuit 601 may be located at the upper portion of the aseptic handle, below the groove 602, and on the outer surface of the inner handle. In order to facilitate the user to hold the region corresponding to the location notch, the surface of the location notch may be a smooth surface or a frosted surface.

In some possible implementation manners, the surface of the location notch may also be provided with a touch screen by which the user's touch operation is sensed.

In some possible implementation manners, in order to facilitate user perception, when the location notch is touched, a prompt such as sound, light or vibration may be given.

In some possible implementation manners, it may also be set to prompt the correspondence relationship between the user holding information and the illumination parameter by voice or image.

According to the embodiment of the present application, the current holding information corresponding to the operation that the finger is located on any location notch is determined as the first preset holding information, and the current holding information corresponding to the operation that no finger is located on any location notch is determined as the second preset holding information. This embodiment facilitates the user to directly control different illumination parameters accurately and conveniently.

It should be understood that, in some possible implementation manners of the present invention, at least two types of preset holding information and the correspondence relationship between each preset holding information and the corresponding illumination parameter thereof are pre-stored. After the holding information that the user holds the handle is determined, the first detection circuit may further determine which type of preset holding information that the holding information is, and the illumination parameter controlled by the current operation can be determined according to the correspondence relationship between the preset holding information and the illumination parameter. Then, the second detection circuit obtains the angle at which the user rotates the aseptic handle and determines, according to the angle, the corresponding adjustment instruction including at least one of increasing the illumination parameter, decreasing the illumination parameter, and keeping the value of the illumination parameter. According to the angle, it can be determined whether the aseptic handle rotates clockwise or counterclockwise, and it can be preset to increase the illumination parameter during the clockwise rotation, decrease the illumination parameter during the counterclockwise rotation, and keep the illumination parameter in the case of no rotation. The second detection circuit may be arranged on the outer surface of the inner handle, or may be arranged on the shell, as long as the angle of rotation of the aseptic handle can be determined, which is not limited.

After the illumination parameter operated by the current holding operation and the angle of rotation of the aseptic handle are obtained, the control circuit can control the illumination parameter operated by the current holding operation. For example, if the illumination parameter corresponding to the current holding operation is the illumination brightness, the control circuit can control the illumination apparatus to increase the brightness when the aseptic handle rotates clockwise, the control circuit can control the illumination apparatus to reduce the brightness when the aseptic handle rotates counterclockwise, and the magnitude or extent of change of the illumination parameter can be determined according to the value of angle.

The illumination parameter corresponding to the preset holding information includes the diameter of illumination spot, the illumination brightness, the color temperature of illumination light, or multiple parameters or settings of certain specific illumination modes. The specific illumination modes are generally a set of certain specific illumination parameters built in the control circuit of the surgical light. The illumination parameter set is usually a set of parameters, such as spot, brightness, shadowless effect, etc., which are set according to the specific requirements of surgery for the illumination, such as different types of surgery or the number of surgeons performing the operation. The user can set, in the surgical light, in advance multiple illumination parameters that he commonly uses or multiple illumination parameters that he most wishes to use during the operation. During the operation, the surgeon can adjust the illumination parameter of the surgical light through different holding gestures, without the need for other persons to adjust according to his requirement, and the surgeon can clearly use the different holding gestures described in the present invention to hold the aseptic handle during the surgical procedure without being distracted from the surgical operation, with a clear, natural and continuous adjustment process. Therefore, according to the technical solution provided in the present application, the surgeon can adjust multiple parameters of the surgical light during the operation more timely and conveniently.

As an improvement, in some possible implementation manners of the present application, the same holding gesture can correspond to different illumination parameters according to the different finger touch positions during holding. In this way, the number of controllable illumination parameters can be increased.

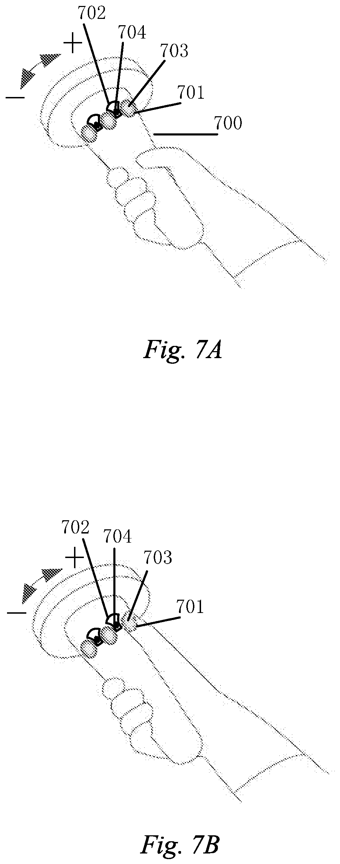

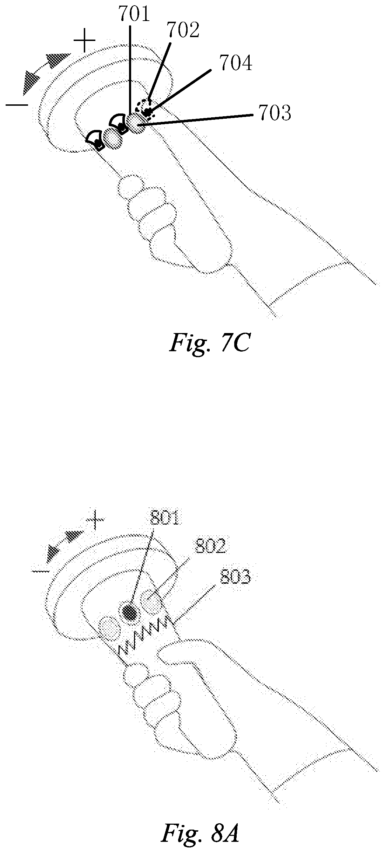

Referring to Figs. 7A, 7B and 7C, the upper circumferential recognition region of the aseptic handle 700 is provided with sensible sub-regions of different shapes. Specifically, the upper outer surface of the aseptic handle is circumferentially provided with location notches 701 and protrusions 702 at intervals. In some possible implementation manners of the present application, the size of the location notch 701 may be adapted to the size of the thumb, and the protrusion 702 may have the size less than that of the thumb and is significantly higher than the location notch 701, which facilitates the user to clearly distinguish his thumb's position. The first detection circuit may comprise a detection circuit 703 for detecting that the user touches the location notch 701, and a detection circuit 704 that the user touches the protrusion 702. It should be noted that it is possible to determine whether the user's holding information is the first preset holding information or the second preset holding information according to the method provided in the previous embodiments, which will not be described here. The difference between this embodiment and the embodiment corresponding to Fig. 6A includes: if the current holding gesture is the gesture that the thumb axially extends along the aseptic handle, the first detection circuit can determine whether the user's finger is located on the location notch 701 or on the protrusion 702, it is determined to perform control corresponding to the first illumination parameter when the finger is located on the location notch 701, it is determined to perform control corresponding to the second illumination parameter when the finger is located on the protrusion 702, and it should be understood that when the user's holding gesture is the second preset holding gesture that the thumb radially extends along the aseptic handle, it corresponds to the third illumination parameter. The first illumination parameter, the second illumination parameter and the third illumination parameter are defined as any three of the multiple illumination parameters of the surgical light, and the controlled illumination parameter may be fixed or may be selected and determined from the multiple illumination parameters as needed before each operation of the surgical light.

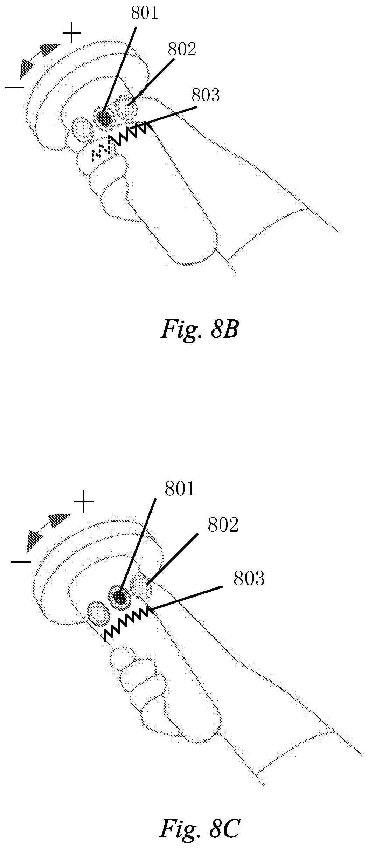

It should be understood that, if the current holding gesture is the second preset holding gesture and the holding position is different, it may also correspond to different illumination parameters. As shown in Figs. 8A and 8B, the user can hold the aseptic handle in the second preset holding gesture. Fig. 8A shows the holding state in which the holding position is located below the region recognition mark 803. Fig. 8B shows the holding state in which the holding position is at least partially located above the region recognition mark 803. The holding states in Figs. 8A and 8B may respectively correspond to different illumination parameters. In order to further increase the number of the controllable illumination parameters, the positions where the user holds the aseptic handle can be further defined. As shown in Figs. 8A, 8B and Fig. 8C, elliptical protrusions 801 and elliptical grooves 802 may be provided above the region recognition mark 803, such that when the user holds the aseptic handle in the first preset holding gesture, the thumb being located at the elliptical protrusion 801 or at the elliptical groove 802 corresponds to different illumination parameters. It should be understood that the controlled illumination parameter may also be determined in conjunction with more position features, and no further examples will be given here.

In some possible implementation manners, the elliptical regions 801 and 802 above the region recognition mark 803 shown in Fig. 8A may be induction regions having the same structure or different structures. In some possible implementation manners, the surface of the region recognition mark may be of a material of touch screen, etc., and the first detection circuit can detect whether the user touches one elliptical induction region or multiple elliptical induction regions. When the user's current holding gesture is determined, the gesture that the user touches one elliptical induction region can be determined as the first preset holding gesture, and the holding gesture that the user touches two or more elliptical induction regions can be determined as the second preset holding gesture. It should be understood that the user's current holding gesture may also be determined in conjunction with other position features, and no further examples will be given here.

The above embodiments are merely used for illustrating rather than limiting the technical solution of the present invention. Although the present invention has been described in detail with reference to the foregoing embodiments, it should be understood by those of ordinary skill in the art that modifications can still be made to the technical solution described in the foregoing embodiments or equivalent substitutions of some technical features thereof is also possible.

The description has been made with reference to various exemplary embodiments herein. However, those skilled in the art will recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions executed on a computer or other programmable data processing apparatus can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing apparatus can provide steps for implementing specified functions.

Although the principles herein have been shown in various embodiments, many modifications of structures, arrangements, ratios, elements, materials, and components that are particularly suitable for specific environments and operating requirements can be made without departing from the scope of the present disclosure.

The foregoing specific description has been described with reference to various embodiments. However, those skilled in the art will recognize that various modifications and changes can be made without departing from the scope of the present disclosure. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising" and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communication connection, functional connection, and/or any other connection.

Those skilled in the art will recognize that many changes can be made to the details of the above-described embodiments without departing from the basic principles of the present invention. Therefore, the scope of the present invention should be determined only by the claims.

## Claims

1. A method for controlling a surgical light, comprising:
obtaining holding information when a user holds an aseptic handle,
determining an illumination parameter corresponding to the holding information according to the holding information;
obtaining an adjustment instruction triggered by the user; and
adjusting the illumination parameter corresponding to the holding information according to the adjustment instruction;
**characterised in that**:
the holding information comprises holding gestures and/or holding positions used by the user when holding the aseptic handle, and different holding gestures and/or different holding positions correspond to different illumination parameters.

2. The method of claim 1, wherein the step of obtaining holding information when a user holds an aseptic handle comprises:
obtaining a touch signal of the user on a recognition region provided on the aseptic handle; and
determining the holding information according to the touch signal.

3. The method of claim 1, wherein the step of obtaining holding information when a user holds an aseptic handle further comprises:
obtaining touch signals of the user on a recognition region and a location region which are provided on the aseptic handle; and
determining the holding information according to the touch signals.

4. The method of claim 1, further comprising:
pre-storing at least two types of preset holding information and a correspondence relationship between each preset holding information and a corresponding illumination parameter.

5. The method of claim 4, wherein the at least two types of preset holding information include a first holding gesture and a second holding gesture, wherein the first holding gesture is a gesture that the user holds the aseptic handle and has a thumb axially extending along the aseptic handle; and the second holding gesture is a gesture that the user holds the aseptic handle and has a thumb radially extending along the aseptic handle.

6. The method of claim 1, wherein the step of obtaining an adjustment instruction triggered by the user comprises:
obtaining an angle at which the user rotates the aseptic handle; and
determining the adjustment instruction according to the angle, the adjustment instruction includes at least one of increasing a value of the illumination parameter, reducing a value of the illumination parameter, and keeping a value of the illumination parameter.

7. The control method of any one of claims 1 to 6, wherein
the illumination parameter includes a diameter of an illumination spot, an illumination brightness, or a color temperature.

8. A surgical light, comprising: a light body (201), an aseptic handle (202), a detection circuit (204, 205), and a control circuit (203), wherein
the light body (201) comprises a shell (2011) and an illumination apparatus (2012) installed in the shell (2011);
the detection circuit (204, 205) is used to obtain holding information when a user holds the aseptic handle (202), and obtain an adjustment instruction triggered by the user; and
the control circuit (203) is used to determine an illumination parameter corresponding to the holding information according to the holding information, and adjust the illumination parameter corresponding to the holding information according to the adjustment instruction obtained by the detection circuit;
**characterised in that**:
the holding information comprises holding gestures and/or holding positions used by the user when holding the aseptic handle, and different holding gestures and/or different holding positions correspond to different illumination parameters.

9. The surgical light of claim 8, wherein the aseptic handle is provided with a recognition region, and
the detection circuit is also used to obtain a touch signal of the user on the recognition region and determine the holding information according to the touch signal.

10. The surgical light of claim 8, wherein the aseptic handle is provided with a recognition region and a location region, and
the detection circuit is also used to obtain touch signals of the user on the recognition region and the location region and determine the holding information according to the touch signals.

11. The surgical light of claim 8, wherein the surgical light further comprises a memory, wherein
the memory is used to store at least two types of preset holding information and a correspondence relationship between each preset holding information and a corresponding illumination parameter.

12. The surgical light of claim 11, wherein
the at least two types of preset holding information include a first holding gesture and a second holding gesture, wherein the first holding gesture is a gesture that the user holds the aseptic handle and has a thumb axially extending along the aseptic handle; and the second holding gesture is a gesture that the user holds the aseptic handle and has a thumb radially extending along the aseptic handle.

13. The surgical light of claim 8, wherein
the detection circuit is also used to obtain an angle at which the user rotates the aseptic handle; and the adjustment instruction is determined according to the angle, the adjustment instruction includes at least one of increasing a value of the illumination parameter, reducing a value of the illumination parameter, and keeping a value of the illumination parameter.

14. The surgical light of any one of claims 8 to 13, wherein
the illumination parameter includes a diameter of an illumination spot, an illumination brightness, or a color temperature.

## Patentansprüche

1. Ein Verfahren zur Steuerung einer chirurgischen Leuchte, umfassend:
Erhalt von Informationen über das Halten, wenn ein Benutzer einen aseptischen Griff hält,
Bestimmen eines Beleuchtungsparameters entsprechend zu der Halteinformation gemäß der Halteinformation;
Erhalten einer vom Benutzer ausgelösten Einstellanweisung; und
Einstellung des Beleuchtungsparameters entsprechend der Halteinformation entsprechend der Anpassung
**gekennzeichnet durch**:
Die Halteinformationen umfassen Haltegesten und/oder Haltepositionen, die der Benutzer verwendet, wenn er den aseptischen Griff hält, und verschiedene Haltegesten und/oder verschiedene Haltepositionen entsprechen verschiedenen Beleuchtungsparametern.

2. Verfahren nach Anspruch 1, wobei der Schritt des Erhaltens von Halteinformationen, wenn ein Benutzer einen aseptischen Griff hält, folgendes umfasst:
Erfassen eines Berührungssignals des Benutzers auf einem Erkennungsbereich, der auf dem aseptischer Griff; und
Bestimmung der Halteinformation anhand des Berührungssignals.

3. Verfahren nach Anspruch 1, wobei der Schritt des Erhaltens von Haltedaten , wenn ein Benutzer einen aseptischen Griff hält, ferner umfasst:
Erfassen von Berührungssignalen des Benutzers an einem Erkennungsbereich und einem Positionsbereich, die auf dem aseptischen Griff bereitgestellt werden; und
Bestimmen der Halteinformationen gemäß den Berührungssignalen.

4. Das Verfahren nach Anspruch 1 umfasst ferner:
Vorspeichern von mindestens zwei Arten von voreingestellten Halteinformationen und einer Korrespondenzbeziehung zwischen jeder voreingestellten Halteinformation und einem entsprechenden Beleuchtungsparameter.

5. Verfahren nach Anspruch 4, wobei die mindestens zwei Arten von voreingestellten Halteinformationen eine erste Haltegeste und eine zweite Haltegeste umfassen, wobei die erste Haltegeste eine Geste ist, bei der der Benutzer den aseptischen Griff hält und einen Daumen hat, der sich axial entlang des aseptischen Griffs erstreckt; und die zweite Haltegeste ist eine
Geste, dass der Benutzer den aseptischen Griff hält und einen Daumen hat, der sich radial entlang des aseptischen Griffs erstreckt.

6. Verfahren nach Anspruch 1, wobei der Schritt des Erhaltens einer durch den Benutzer ausgelösten Einstellanweisung folgendes umfasst:
Erhalten eines Winkels, in dem der Benutzer den aseptischen Griff dreht; und
Bestimmen der Einstellanweisung entsprechend dem Winkel, wobei die Einstellanweisung mindestens eine der folgenden Optionen umfasst: Erhöhen eines Wertes des Beleuchtungsparameters, Verringern eines Wertes des Beleuchtungsparameters und Beibehalten eines Wertes des Beleuchtungsparameters.

7. Das Kontrollverfahren nach einem der Ansprüche 1 bis 6, wobei der Beleuchtungsparameter einen Durchmesser eines Beleuchtungsflecks, eine Beleuchtungshelligkeit oder eine Farbtemperatur umfasst.

8. eine chirurgische Leuchte, umfassend: einen Beleuchtungskörper (201), einen aseptischen Griff (202), eine Erfassungsschaltung (204, 205) und eine Steuerschaltung (203), wobei
der Beleuchtungskörper (201) ein Gehäuse (2011) und eine Beleuchtungsvorrichtung (2012) umfasst, die in dem Gehäuse (2011) installiert ist;
die Erkennungsschaltung (204, 205) wird verwendet, um Halteinformationen zu erhalten, wenn ein Benutzer den aseptischen Griff (202) hält und eine vom Benutzer ausgelöste Einstellanweisung erhält, und
die Steuerschaltung (203) zur Bestimmung eines Beleuchtungsparameters verwendet wird, der die Betriebsinformation gemäß der Betriebsinformation entspricht , und Einstellen des Beleuchtungsparameters , der mit der Halteinformation korrespondiert, gemäß der von der Erfassungsschaltung erhaltenen Einstellanweisung;
**dadurch gekennzeichnet**:
Die Halteinformationen umfassen Haltegesten und/oder Haltepositionen, die der Benutzer verwendet, wenn er den aseptischen Griff hält, und verschiedene Haltegesten und/oder verschiedene Haltepositionen entsprechen verschiedenen Beleuchtungsparametern.

9. Die chirurgische Leuchte nach Anspruch 8, wobei der aseptische Griff mit folgenden Merkmalen einer Anerkennungsregion versehen ist, und
die Erkennungsschaltung auch verwendet wird, um ein Berührungssignal des Benutzers auf dem Erkennungsbereich zu erhalten und die Halteinformation entsprechend dem Berührungssignal zu bestimmen.

10. Die Chirurgische Leuchte nach Anspruch 8, wobei der aseptische Griff mit einem Erkennungsbereich und einem Ortungsbereich versehen ist, und
der Erkennungskreislauf wird auch verwendet, um Berührungssignale des Benutzers im Erkennungsbereich und im Standortbereich zu erhalten und die Halteinformationen gemäß den Berührungssignalen zu bestimmen.

11. Chirurgische Leuchte nach Anspruch 8, wobei die chirurgische Leuchte außerdem einen Speicher umfasst, wobei
der Speicher zur Speicherung von mindestens zwei Arten von voreingestellten Betriebsinformationen und eine Korrespondenzbeziehung zwischen jeder voreingestellten Information und einem entsprechenden Beleuchtungsparameter dient

12. Die chirurgische Leuchte nach Anspruch 11, wobei die mindestens zwei Arten von voreingestellten Halteinformationen eine erste Haltegeste und eine zweite Haltegeste umfassen, wobei die erste Haltegeste eine Geste ist, dass der Benutzer den aseptischen Griff hält und sich ein Daumen axial entlang des aseptischen Griffs erstreckt; und die zweite Haltegeste ist eine Geste, dass der Benutzer den aseptischen Griff hält und sich ein Daumen radial entlang des aseptischen Griffs erstreckt.

13. Die chirurgische Leuchte nach Anspruch 8, wobei die Erfassungsschaltung auch verwendet wird, um einen Winkel zu erhalten, in dem der Benutzer den aseptischen Griff dreht; und die Einstellanweisung wird entsprechend dem Winkel bestimmt, der Einstellbefehl mindestens einen der folgenden Werte umfasst: Erhöhen eines Wertes des Beleuchtungsparameters, Verringern eines Wertes des Beleuchtungsparameters und Beibehalten von eines Wertes des Beleuchtungsparameters.

14. Die chirurgische Leuchte nach einem der Ansprüche 8 bis 13, wobei der Beleuchtungsparameter einen Durchmesser eines Beleuchtungsflecks, einer Beleuchtungshelligkeit oder einer Farbtemperatur umfasst.

## Revendications

1. Procédé de commande de lampe chirurgicale, comprenant les étapes consistant à :
obtenir des informations de tenue lorsqu'un utilisateur tient une poignée aseptique ;
déterminer un paramètre d'éclairage correspondant aux informations de tenue selon les informations de tenue ;
obtenir une instruction de réglage initiée par l'utilisateur ; et
régler le paramètre d'éclairage correspondant aux informations de tenue selon l'instruction de réglage ;
**caractérisé en ce que** :
les informations de tenue comprennent des gestes de tenue et/ou des positions de tenue utilisés par l'utilisateur lorsqu'il tient la poignée aseptique, et différents gestes de tenue et/ou différentes positions de tenue correspondent à différents paramètres d'éclairage.

2. Procédé selon la revendication 1, dans lequel l'étape consistant à obtenir des informations de tenue lorsqu'un utilisateur tient une poignée aseptique comprend :
le fait d'obtenir un signal de contact de l'utilisateur sur une zone de reconnaissance prévue sur la poignée aseptique ; et
le fait de déterminer les informations de tenue selon le signal de contact.

3. Procédé selon la revendication 1, dans lequel l'étape consistant à obtenir des informations de tenue lorsqu'un utilisateur tient une poignée aseptique comprend en outre :
le fait d'obtenir des signaux de contact de l'utilisateur sur une zone de reconnaissance et une zone de position lesquelles sont prévues sur la poignée aseptique ; et
le fait de déterminer les informations de tenue selon les signaux de contact.

4. Procédé selon la revendication 1, comprenant en outre :
le fait de pré-mémoriser au moins deux types d'informations de tenue prédéfinies et une relation de correspondance entre chaque information de tenue prédéfinie et un paramètre d'éclairage correspondant.

5. Procédé selon la revendication 4, dans lequel les au moins deux types d'informations de tenue prédéfinies incluent un premier geste de tenue et un second geste de tenue, où le premier geste de tenue est un geste où l'utilisateur tient la poignée aseptique et a son pouce s'étendant axialement le long de la poignée aseptique ; et le second geste de tenue est un geste où l'utilisateur tient la poignée aseptique et a son pouce s'étendant radialement le long de la poignée aseptique.

6. Procédé selon la revendication 1, dans lequel l'étape consistant à obtenir une instruction de réglage initiée par l'utilisateur comprend :
le fait d'obtenir un angle selon lequel l'utilisateur tourne la poignée aseptique ; et
le fait de déterminer l'instruction de réglage selon l'angle, l'instruction de réglage inclut au moins une instruction parmi une augmentation d'une valeur du paramètre d'éclairage, une diminution d'une valeur du paramètre d'éclairage et une conservation d'une valeur du paramètre d'éclairage.

7. Procédé de commande selon l'une quelconque des revendications 1 à 6, dans lequel le paramètre d'éclairage inclut un diamètre d'un point d'éclairage, une luminosité d'éclairage, ou une température de couleur.

8. Lampe chirurgicale, comprenant : un corps lumineux (201), une poignée aseptique (202), un circuit de détection (204, 205), et un circuit de commande (203), où
le corps lumineux (201) comprend une enveloppe (2011) et un dispositif d'éclairage (2012) installé à l'intérieur de l'enveloppe (2011) ;
le circuit de détection (204, 205) est utilisé pour obtenir des informations de tenue lorsqu'un utilisateur tient la poignée aseptique (202), et obtenir une instruction de réglage initiée par l'utilisateur ; et
le circuit de commande (203) est utilisé pour déterminer un paramètre d'éclairage correspondant aux informations de tenue selon les informations de tenue, et régler le paramètre d'éclairage correspondant aux informations de tenue selon l'instruction de réglage obtenue par le circuit de détection ;
**caractérisée en ce que** :
les informations de tenue comprennent des gestes de tenue et/ou des positions de tenue utilisés par l'utilisateur lorsqu'il tient la poignée aseptique, et différents gestes de tenue et/ou différentes positions de tenue correspondent à différents paramètres d'éclairage.

9. Lampe chirurgicale selon la revendication 8, dans laquelle la poignée aseptique est dotée d'une zone de reconnaissance, et
le circuit de détection est également utilisé pour obtenir un signal de contact de l'utilisateur sur la zone de reconnaissance et déterminer les informations de tenue selon le signal de contact.

10. Lampe chirurgicale selon la revendication 8, dans laquelle la poignée aseptique est dotée d'une zone de reconnaissance et d'une zone de position, et
le circuit de détection est également utilisé pour obtenir des signaux de contact de l'utilisateur sur la zone de reconnaissance et sur la zone de position et déterminer les informations de tenue selon les signaux de contact.

11. Lampe chirurgicale selon la revendication 8, où la lampe chirurgicale comprend en outre une mémoire, où
la mémoire est utilisée pour mémoriser au moins deux types d'informations de tenue prédéfinies et une relation de correspondance entre chaque information de tenue prédéfinie et un paramètre d'éclairage correspondant.

12. Lampe chirurgicale selon la revendication 11, dans laquelle les au moins deux types d'informations de tenue prédéfinies incluent un premier geste de tenue et un second geste de tenue, où le premier geste de tenue est un geste où l'utilisateur tient la poignée aseptique et a son pouce s'étendant axialement le long de la poignée aseptique ; et le second geste de tenue est un geste où l'utilisateur tient la poignée aseptique et a son pouce s'étendant radialement le long de la poignée aseptique.

13. Lampe chirurgicale selon la revendication 8, dans laquelle le circuit de détection est également utilisé pour obtenir un angle selon lequel l'utilisateur tourne la poignée aseptique ; et l'instruction de réglage est déterminée selon l'angle, l'instruction de réglage inclut au moins une instruction parmi une augmentation d'une valeur du paramètre d'éclairage, une diminution d'une valeur du paramètre d'éclairage et une conservation d'une valeur du paramètre d'éclairage.

14. Lampe chirurgicale selon l'une quelconque des revendication 8 à 13, dans laquelle le paramètre d'éclairage inclut un diamètre d'un point d'éclairage, une luminosité d'éclairage, ou une température de couleur.
